# EUROPÄISCHE PATENTSCHRIFT

(11) **EP 0 842 145 B1**
(45) Veröffentlichungstag und Bekanntmachung des Hinweises auf die Patenterteilung: **19.07.2000**
(21) Anmeldenummer: 96927595.7
(22) Anmeldetag: 24.07.1996
(51) Int. Cl.: C07C 253/30, C07B 37/04

(54) **VERFAHREN ZUR DURCHFÜHRUNG VON KREUZKUPPLUNGSREAKTIONEN**
METHOD OF CARRYING OUT CROSS-COUPLING REACTIONS
PROCEDE DE MISE EN UVRE DE REACTIONS DE COUPLAGES CROISES

(30) Priorität: 25.07.1995 DE 19527118; 25.09.1995 DE 19535528; 17.05.1996 DE 19620023
(43) Veröffentlichungstag der Anmeldung: 20.05.1998
(73) Patentinhaber: Clariant GmbH, 65929 Frankfurt am Main (DE)
(72) Erfinder: HABER, Steffen, D-61462 Königstein (DE); KLEINER, Hans-Jerg, D-61476 Kronberg (DE)
(86) Internationale Anmeldenummer: EP9603267
(87) Internationale Veröffentlichungsnummer: WO9705104

(56) Entgegenhaltungen:
- EP-A- 0 571 770
- WO-A-94/10105
- DE-A- 4 340 490
- JOURNAL OF ORGANIC CHEMISTRY, Bd. 59, Nr. 20, 1994, EASTON US, Seiten 6095-6097, XP000469000 S. W. WRIGHT ET AL.: "Fluoride-mediated boronic acid coupling reactions"
- JOURNAL OF MEDICINAL CHEMISTRY, Bd. 34, Nr. 9, 1991, WASHINGTON US, Seiten 2919-22, XP002018179 N. B. MANTLO ET AL.: "Potent, orally active imidazo[4,5-b]pyridine-based angiotensin II receptor antagonists"

## Beschreibung

Die Erfindung betrifft ein Verfahren zur Herstellung mehrkerniger aromatischer Verbindungen durch Kreuzkupplungsreaktion von aromatischen Borverbindungen und aromatischen Halogenverbindungen oder Perfluoralkylsulfonaten unter Nickel- oder Palladiumkatalyse.

Kreuzkupplungsreaktionen von aromatischen Borverbindungen, wie Boronsäuren, und aromatischen Halogenverbindungen oder Perfluoralkylsulfonaten werden seit einigen Jahren in steigendem Umfang zum Aufbau mehrkerniger aromatischer Systeme benutzt. Beispielsweise dienen solche Verfahren zur Herstellung von pharmazeutischen Wirkstoffen und Komponenten von Flüssigkristallmischungen.

Die üblicherweise verwendeten Katalysatoren, wie Pd[P(Ph₃)]₄ oder PdCl₂(4PPh₃)4NaBH₄, liefern aber nur mit Brom- oder lodaromaten die Kupplungsprodukte in nennenswerten Ausbeuten. Die hohen Kosten dieser Ausgangsverbindungen erschweren eine wirtschaftliche Überführung der Prozesse in einen größeren Produktionsmaßstab.

So wird z.B. in S.W.Wright et al., J.Org.Chem., Vol.59, No. 20, 1994, S. 6095-6097 ein Verfahren zur Flourid-katalysierten Boronsäure-Kreuzkupplung in DMSO als Lösemittel beschrieben. Die Ausbeuten sind jedoch nicht zufriedenstellend.

Will man auf die kostengünstigeren Chloraromaten als Ausgangsverbindungen zurückgreifen, müssen, wie in der DE-A- 43 40 490 beschrieben, ein Palladiumkatalysator und lipophile, aliphatische Phosphanliganden eingesetzt werden.

Solche Phosphane sind aber nicht nur aufwendig herzustellen und stark oxidationsempfindlich, sie können zudem auch nicht wiederverwendet werden.

Weiterhin sind, wenn der Phosphanligand nicht auch Cycloalkylgruppen enthält, die Ausbeuten des in DE-A 43 40 490 beschriebenen Verfahrens durchaus noch verbesserungsfähig.

Es war daher wünschenswert, ein Verfahren zu entwickeln, das die Kupplung von aromatischen Chlorverbindungen mit aromatischen Borverbindungen in hohen Ausbeuten ermöglicht, ohne daß lipophile, aliphatische Phosphane, die Cycloalkylgruppen enthalten, eingesetzt werden müssen.

Es wurde nun überraschend gefunden, daß Chlor- und andere Halogen- oder Perfluoralkylsulfonat-substituierte Aromaten und aromatische Borverbindungen in Gegenwart eines phosphorhaltigen Komplexliganden unter Palladium- oder Nickelkatalyse in hohen Ausbeuten gekuppelt werden können, wenn bei der Reaktion ein oder mehrere mehrwertige Alkohole, Sulfoxide oder Sulfone zugesetzt werden.

Gegenstand der Erfindung ist daher ein Verfahren zur Herstellung mehrkerniger aromatischer Verbindungen, dadurch gekennzeichnet, daß man
a) eine aromatische Borverbindung mit
b) einer aromatischen Halogenverbindung oder einem aromatischen Perfluoralkylsulfonat in Gegenwart
c) einer Base aus der Gruppe der Alkali- und Erdalkalimetallhydroxide, Alkali- und Erdalkalimetallcarbonate, Alkalimetallhydrogencarbonate, Alkali- und Erdalkalimetallacetate, Alkali- und Erdalkalimetallalkoholate, sowie primäre, sekundäre und tertiäre Amine,
d) einer Nickel- oder Palladiumverbindung als Katalysator,
e) eines phosphorhaltigen Liganden und
f) eines mehrwertigen Alkohols, eines Sulfoxides oder Sulfons umsetzt.

Durch das erfindungsgemäße Verfahren lassen sich mehrkernige aromatische Verbindungen ökonomisch in sehr guten Ausbeuten und gleichzeitig sehr hoher Reinheit, insbesondere ohne Verunreinigung durch Phosphanliganden herstellen.

Es eignet sich auch gut zur Kupplung von Chloraromaten und bietet daher beträchtliche ökonomische Vorteile.

Das Verfahren ist chemoselektiv, so daß selbst elektrophile Gruppen, wie Ester und Nitrile, den Verlauf der Reaktion nicht beeinträchtigen.

Bevorzugte mehrwertige Alkohole sind solche, die wasserlöslich sind. Besonders bevorzugt sind Glykole, Glycerin, Oligoglyceride, die auch teilverestert sein können, Di-, Tri- und Tetraethylenglykol oder auch Polyethylenglykole der allgemeinen Formel (I), 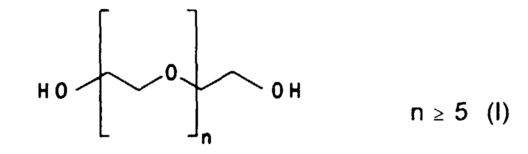 mehrwertige Alkan- oder Alkenole, wie 1,4-Butandiol, 1,3-Propandiol, 1,2-Propandiol, Pentaerythrit, 2-Ethylhexan-1,3-diol, 2-(Hydroxymethyl)-2-methyl-1,3-propandiol, 2-Methyl-2,4-pentandiol, 1,4-cis-Butendiol, mehrwertige Cycloalkanole, wie Cyclohexandiol, mehrwertige, Arylgruppen enthaltende Alkanole, wie 1-Phenyl-1,2-ethandiol, mehrwertige Aminoalkohole, wie Diethanolamin, Triethanolamin, 2-Amino-2-methyl-1,3-propandiol, 3-(Aminomethyl)-1,2-propandiol, 3-Amino-1,2-propandiol, 2-Amino-1,3-propandiol-oxalat, 3-(Diethylamino)-1,2-propandiol, Ethylendiamin-N,N,N',N'-tetra-2-propandiol, mehrwertige Iminoalkohole, wie N-Butyl- und N-tert.-Butyl-2,2'-iminodiethanol, 1,1'-lminodi-3-propanol, N-Methyl-2,2'-iminodiethanol, N-Phenyl-2,2'-iminodiethanol, oder auch Verbindungen wie 1,1',1"-Nitrilo-tri-2-propanol, 1,3,5-Tri-(2-hydroxyethyl)-isocyanursäure und Dihydroxyaceton.

Ganz besonders bevorzugt sind Glykol, Glycerin, 1,4-Butandiol, 1,2-Propandiol, Triethylenglykol, Diethylenglykol, Diethanolamin und Triethanolamin und davon insbesondere Glykol, Glycerin, 1,4-Butandiol und 1,2-Propandiol.

Es können natürlich auch mehrere mehrwertige Alkohole eingesetzt werden.

Bevorzugte Sulfoxide bzw. Sulfone sind solche der allgemeinen Formel (II): 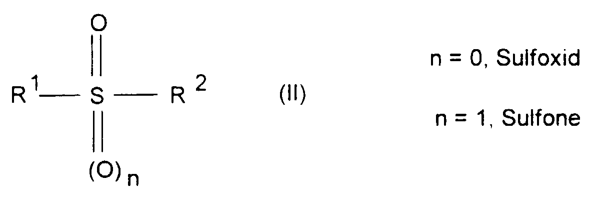 R¹, R² sind aliphatische oder aromatische Kohlenwasserstoffe, die gegebenenfalls substituiert oder miteinander verknüpft sein können.

Besonders bevorzugte Sulfoxide sind Dimethylsulfoxid (DMSO), Diphenylsulfoxid, Methylphenylsulfoxid und Dibenzylsulfoxid.

Besonders bevorzugte Sulfone sind Bis(4-hydroxyphenyl)sulfon, Bis-(4-aminophenyl)sulfon (Dapson), Bis(3-aminophenyl)sulfon, Dimethylsulfon, Diphenylsulfon, Sulfolan, 3-Sulfolen.

Bevorzugt werden wasserlösliche Sulfoxide oder Sulfone eingesetzt. Besonders bevorzugte wasserlösliche Sulfoxide oder Sulfone sind DMSO und Sulfolan.

Es können natürlich auch mehrere Sulfoxide oder Sulfone oder deren Gemische, gegebenenfalls auch mit mehrwertigen Alkoholen, eingesetzt werden.

Für das Verfahren geeignet sind auch Sulfonamide und aliphatische bzw. aromatische Sulfonate.

Dienen der mehrwertige, wasserlösliche Alkohol bzw. das Sulfoxid oder Sulfolan nicht als alleiniges Lösungsmittel, werden sie vorzugsweise in einem Gewichtsverhältnis von 0,1 bis 10 000, bezogen auf den Katalysator, zugesetzt.

Als phosphorhaltige Liganden eignen sich vorzugsweise Tri-n-alkylphosphane, Triarylphosphane, Dialkylarylphosphane, Alkyldiarylphosphane und Heteroarylphosphane, wie Tripyridylphosphan und Trifurylphosphan, wobei die drei Substituenten am Phosphor gleich oder verschieden, chiral oder achiral sein können und wobei einer oder mehrere der Substituenten die Phosphorgruppen mehrerer Phosphane verknüpfen können und wobei ein Teil dieser Verknüpfung auch ein oder mehrere Metallatome sein können, Phosphite, Phosphinigsäureester und Phosphonigsäureester, Phosphole, Dibenzophosphole und Phosphoratome enthaltende cyclische bzw. oligo- und polycyclische Verbindungen.

Besonders bevorzugt sind Phosphane, die mindestens eine Arylgruppe am Phosphor enthalten, d.h. Triarylphosphane, Diarylalkylphosphane und Dialkylarylphosphane, und Phosphite.

Besonders bevorzugt in Systemen, die eine wäßrige Phase enthalten, sind wasserlösliche Phosphanliganden, die mindestens eine Arylgruppe enthalten.

Ganz besonders beovrzugt sind Triarylphosphane.

Insbesondere bevorzugt sind:

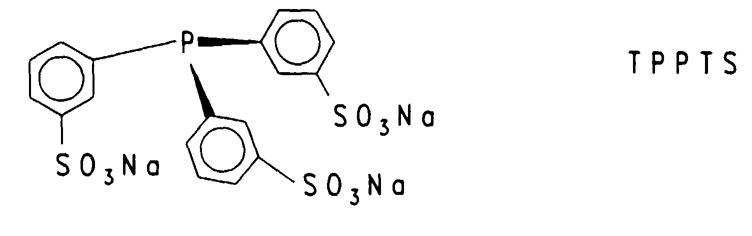

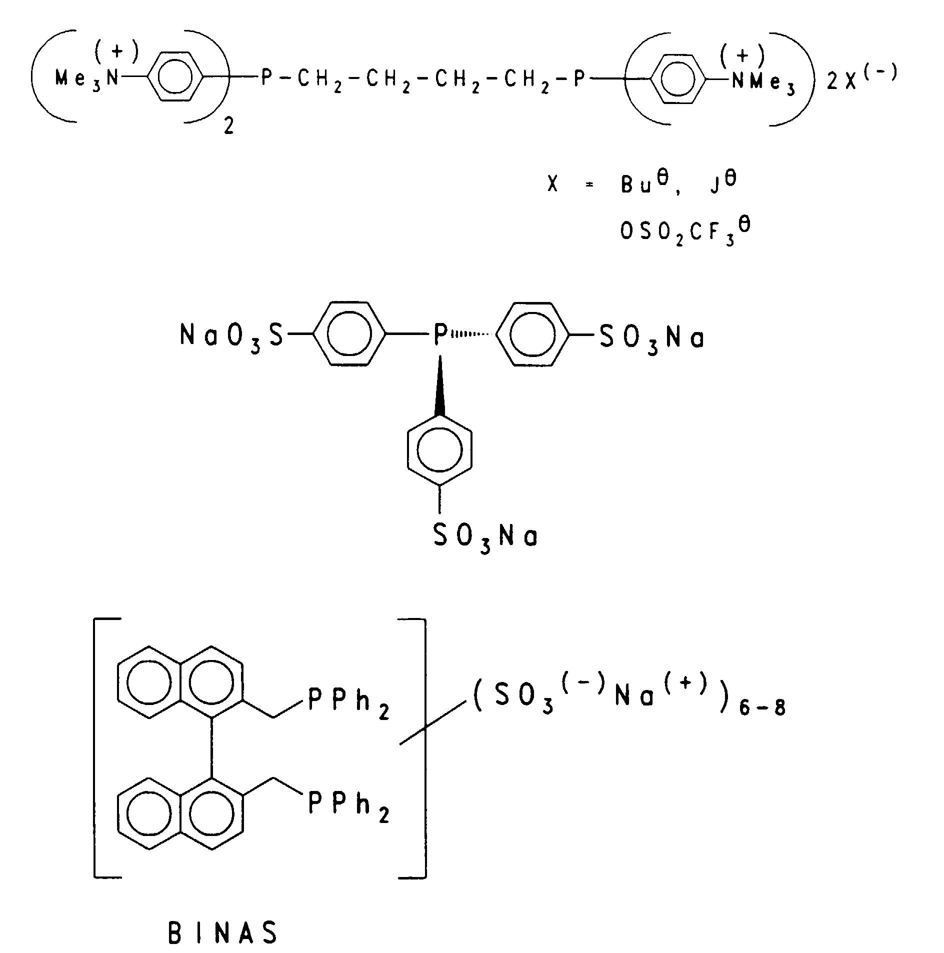

Es können natürlich auch mehrere phosphorhaltige Liganden eingesetzt werden.

Die erfindungsgemäß eingesetzten phosphorhaltigen Liganden sind an sich bekannt. Teilweise sind es kommerzielle Produkte oder sie sind mit ihrer Synthese beispielsweise in Houben-Weyl, Methoden der Organischen Chemie, Georg-Thieme-Verlag, Stuttgart, beschrieben.

Wasserlösliche Liganden können beispielsweise nach W.A. Herrmann und C.W. Kohlpainter, Angew. Chem. Int. Ed. Engl. 1993, 32, 1524 oder der dort zitierten Literatur hergestellt werden. Die Herstellung von BINAS ist in der EP-A 0 571 819 bzw.US-A 5,347,045 beschrieben.

Der phosphorhaltige Ligand wird bei dem erfindungsgemäßen Verfahren mit einem Anteil von 0,001 bis 20 Mol-%, bevorzugt 0,01 bis 10 Mol-%, besonders bevorzugt 0,05 bis 6 Mol-%, insbesondere bevorzugt 0,1 bis 6 Mol-%, bezogen auf die aromatische Halogenverbindung oder das aromatische Perfluoralkylsulfonat, eingesetzt.

Als Katalysatoren werden Palladiumverbindungen oder Nickelverbindungen eingesetzt.

Bevorzugt sind Palladiumverbindungen, in denen das Palladium in der Oxidationsstufe (0) oder (II) vorliegt, wie Palladiumketonate, Palladiumacetylacetonate, Nitrilpalladiumhalogenide, Palladiumhalogenide, Allylpalladiumhalogenide und Palladiumbiscarboxylate, besonders bevorzugt Palladiumketonate, Palladiumacetylacetonate, Palladium(ll)halogenide, η-³-Allylpalladiumhalogenid Dimere und Palladiumbiscarboxylate. Ganz besonders bevorzugt sind Palladiumbisacetylacetonat, Bis(benzonitril)palladiumdichlorid, PdCl₂, Na₂PdCl₄, Na₂Pd₂Cl₆, Bis(acetonitril)palladiumdichlorid, Palladium-II-acetat, Palladium-II-propionat und Palladium-II-butanoat.

Die Palladiumverbindung kann auch in situ erzeugt werden, beispielsweise Palladium(II)acetat durch Zugabe von Palladium(II)chlorid und Natriumacetat.

Der Katalysator kann den erfindungsgemäß eingesetzten phosphorhaltigen Liganden bereits enthalten, der Ligand kann dem Reaktionsgemisch aber auch separat zugesetzt werden.

Zur Durchführung des erfindungsgemäßen Verfahrens ist es bevorzugt, eine Palladium-Verbindung in einem mehrwertigen Alkohol, Sulfoxid oder Sulfolan, vorzugsweise DMSO oder Glykol zu lösen, mit dem phosphorhaltigen Liganden oder einer Lösung desselben zu versetzen und die so gebildete Katalysatorlösung zu den übrigen Reaktanden zu geben.

Ebenso bevorzugt ist es, eine Palladium-Verbindung in einem mehrwertigen Alkohol, Sulfoxid oder Sulfolan, vorzugsweise DMSO oder Glykol, zu lösen, diese Lösung mit den übrigen Reaktanden zu versetzen und anschließend den phosphorhaltigen Liganden oder eine Lösung desselben zuzugeben.

Besonders geeignete Ausgangsverbindungen zur Herstellung des Katalysators nach diesen beiden Verfahren sind Pd(II)Cl₂/3 NaOAc, Pd(ac)₂, K₂PdCl₄, Na₂PdCl₄, K₂PdCl₆ und Na₂PdCl₆.

Der Katalysator wird bei dem erfindungsgemäßen Verfahren mit einem Anteil von 0,001 bis 10 Mol-%, bevorzugt 0,01 bis 5 Mol-%, besonders bevorzugt 0,05 bis 3 Mol-%, insbesondere bevorzugt 0,05 bis 1,5 Mol-%, bezogen auf die aromatische Halogenverbindung oder das aromatische Perfluoralkylsulfonat, eingesetzt.

Besonders bevorzugte Basen sind Alkali- und Erdalkalimetallhydroxide, Alkali- und Erdalkalimetallcarbonate und Alkalimetallhydrogencarbonate. Insbesondere bevorzugt sind Alkalimetallhydroxide, wie Natriumhydroxid und Kaliumhydroxid, sowie Alkalimetallcarbonate und Alkalimetallhydrogencarbonate, wie Lithiumcarbonat, Natriumcarbonat und Kaliumcarbonat. Bei Verwendung fester Basen, wie Na₂CO₃, wird der mehrwertige Alkohol oder das Sulfoxid vorzugsweise in größeren Mengen oder als Lösungsmittel eingesetzt, um eine geeignete Suspendierung der Base und damit ein rührfähiges Gemisch zu erreichen.

Es können natürlich auch mehrere Basen zugesetzt werden.

Die Base wird bei dem erfindungsgemäßen Verfahren bevorzugt mit einem Anteil von 100 bis 1000 Mol-%, besonders bevorzugt 100 bis 500 Mol-%, ganz besonders bevorzugt 100 bis 400 Mol-%, insbesondere 100 bis 290 Mol-%, bezogen auf die aromatische Borverbindung, eingesetzt.

Bevorzugte Ausgangsverbindungen für das erfindungsgemäße Verfahren sind zum einen aromatische Borverbindungen der Formel (III),

Aryl―BQ₁Q₂ (III)

worin
- Aryl: ein aromatischer Rest ist und
- Q₁, Q₂: gleich oder verschieden -OH, C₁-C₄-Alkoxy, C₁-C₄-Alkyl, Phenyl, das gegebenenfalls durch C₁-C₄-Alkyl, C₁-C₄-Alkoxy oder Halogen substituiert sein kann, oder Halogen bedeuten oder Q₁ und Q₂ zusammen bilden eine C₁-C₄-Alkylendioxy-Gruppe, eine Methylengruppe, die gegebenenfalls durch eine oder zwei C₁-C₄-Alkylgruppen substituiert sein kann, oder Q₁ und Q₂ und das Boratom zusammen sind Teil eines Boroxinrings der Formel (IV): 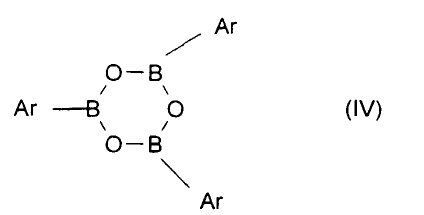
- Aryl: bedeutet bevorzugt einen Phenyl-, Naphthyl-, Pyrimidyl-, Pyridin-, Pyrazin-, Pyradazin-, 1,3-Thiazol, 1,3,4-thiadiazol- oder Thiophenylrest, die alle gegebenenfalls substituiert sein können, beispielsweise mit Halogen, Cyano, Alkyl oder Alkoxygruppen.
- Q₁, Q₂: sind vorzugsweise gleich oder verschieden -OH, C₁-C₄-Alkoxy oder Halogen oder Q₁ und Q₂ zusammen bilden eine C₁-C₄-Alkylendioxy-Gruppe oder Q₁ und Q₂ und das Boratom zusammen sind teil eines Boroxinrings der Formel (IV):

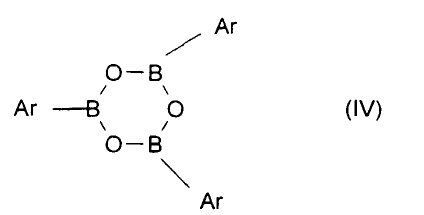

Besonders bevorzugt bedeutet Aryl eine unsubstituierte oder substituierte Phenyl- oder Naphthylgruppe.

Ganz besonders bevorzugte aromatische Borverbindungen sind

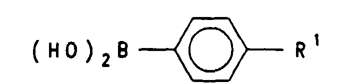

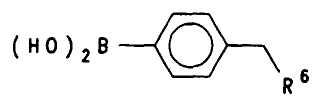

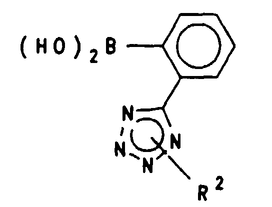

Ganz besonders bevorzugte Verbindungen sind: 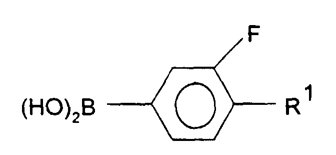 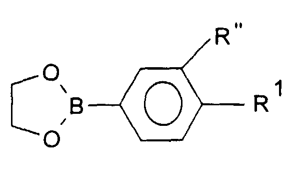 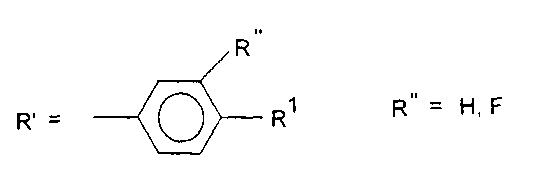 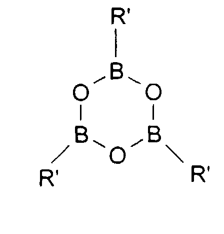 wobei R¹ und R² Benzyloxy, H, Methyl, Ethyl, Propyl, Butyl, Pentyl, Hexyl, Heptyl, Octyl, Nonyl, Decyl, Undecyl, Dodecyl, Tridecyl, Tetradecyl und Pentadecyl, sowie Methoxy, Ethoxy, Propoxy, Butoxy, Pentoxy, Hexoxy, Heptoxy, Octoxy, Nonoxy, Decoxy, Undecoxy, Dodecoxy, Tridecoxy, Tetradecoxy, Pentadecoxy, CPh₃ und SiMe₂tBu, und
R⁶ Imidazol, Chinolin, Isochinolin, Dihydropyridin oder Pyrazol, die alle gegebenenfalls substituiert sein können,
bedeuten.
Insbesondere bevorzugt ist p-Toluolboronsäure.

Die verwendeten aromatischen Borverbindungen sind entweder bekannt oder können nach an sich bekannten Methoden, wie beispielsweise in Houben Weyl Methoden der Organischen Chemie, Georg Thieme-Verlag, Stuttgart, Band 13/3a beschrieben, hergestellt werden. So ist es beispielsweise möglich, aus aromatischen Alkalimetall- und Magnesiumverbindungen durch Umsetzung mit Trialkoxyboranen und anschließender Hydrolyse Boronsäuren zu erhalten.

Die zweite Klasse von Ausgangsverbindungen für das erfindungsgemäße Verfahren sind aromatische Verbindungen der Formel (V)

Aryl - X (V)

wobei
- Aryl: einen aromatischen Rest und
- X: Cl, Br, l oder ein Perfluoralkylsulfonat bedeutet.
- X: bedeutet vorzugsweise Cl.
- Aryl: bedeutet vorzugsweise einen unsubstituierten oder substitueirten Phenyl-, Naphthyl-, Pyridin-, Pyrimidin-, Pyrazin-, Pyridazin-, 1,3-Thiazol-, 1,3,4-Thiadiazol- oder Thiophenrest, wobei der oder die Substituenten beispielsweise Halogen, CN, Alkyl-, Alkoxy- oder weitere Arylgruppen sind.

Besonders bevorzugte Verbindungen der Formel (V) sind 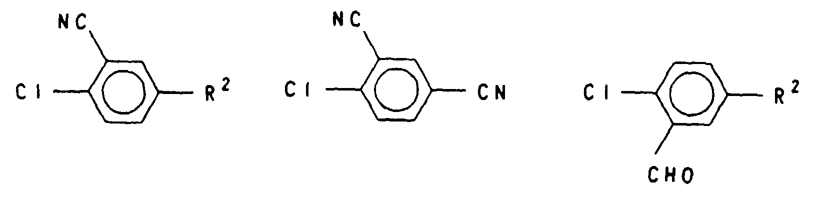 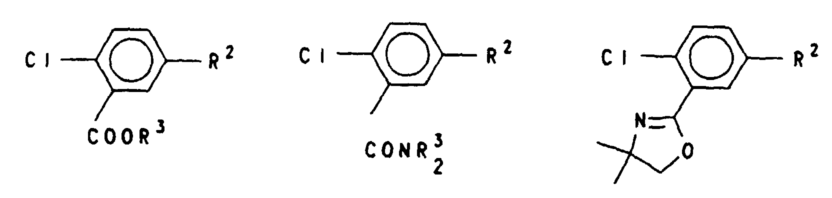 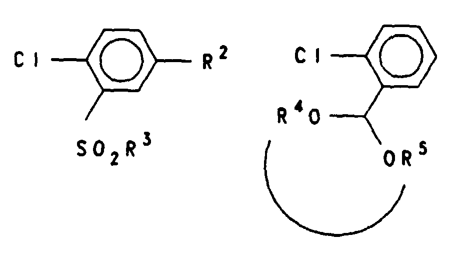 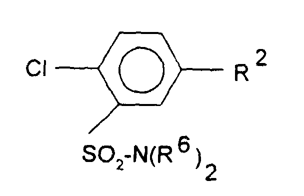 wobei R² und R³ Benzyloxy, H, Methyl, Ethyl, Propyl, Butyl, Pentyl, Hexyl, Heptyl, Octyl, Nonyl, Decyl, Undecyl, Dodecyl, Tridecyl, Tetradecyl und pentadecyl, sowie Methoxy, Ethoxy, propoxy, Butoxy, Pentoxy, Hexoxy, Heptoxy, Octoxy, Nonoxy, Decoxy, Undecoxy, Dodecoxy, Tridecoxy, Tetradecoxy, Pentadecoxy, CPh₃ und SiMe₂Bu; R⁴, R⁵ Methyl, Ethyl, Propyl, Butyl, Pentyl, Hexyl, Heptyl, Octyl, Nonyl, Decyl, Undecyl, Dodecyl, Tridecyl, Tetradecyl, Pentadecyl oder R⁴ und R⁵ zusammen auch -(CH₂)₂- oder -(CH₂)₃- bedeuten.

Ganz besonders bevorzugt ist 2-Chlorbenzonitril.

Die verwendeten aromatischen Halogenverbindungen und Perfluoralkylsulfonate sind entweder bekannt oder können nach bekannten Methoden, wie beispielsweise in Houben Weyl, Methoden der Organischen Chemie,
Georg Thieme Verlag, Stuttgart, Band 5/3 und 5/4 beschrieben, hergestellt werden. Beispielsweise lassen sich aromatische Halogenide dadurch erhalten, daß man in einem entsprechenden Diazoniumsalz die Diazoniumgruppe durch Chlor, Brom oder lod ersetzt.

Des weiteren lassen sich Hydroxy-Stickstoffheterocyclen mit Hilfe von Phosphortrihalogeniden und Phosphoroxytrihalogeniden in die entsprechenden Halogenide überführen.

Zur Durchführung des erfindungsgemäßen Verfahrens werden die Edukte, die Base, die Palladiumverbindung oder die Nickelverbindung und der phosphorhaltige Ligand nach den oben angegebenen Varianten gemischt und bei einer Temperatur von 0 bis 200°C, bevorzugt 30 bis 170°C, besonders bevorzugt 50 bis 150°C, über einen Zeitraum von 1 bis 100 h, vorzugsweise 5 bis 70 h, besonders bevorzugt 5 bis 50 h umgesetzt.

Die Aufarbeitung erfolgt nach bekannten, dem Fachmann geläufigen Methoden. Beispielsweise kann das Produkt durch Extraktion oder Ausfällen vom Reaktionsgemisch abgetrennt und anschließend nach dem jeweiligen Produkt angemessenen Methoden, wie Umkristallisation, Destillation, Sublimation, Zonenschmelzen, Schmelzkristallisation oder Chromatographie, weiter aufgereinigt werden.

Werden als Ausgangsverbindungen zweifach funktionalisierte aromatische Borverbindungen, wie Bisboronsäuren, und aromatische Halogenverbindungen oder Perfluoralkylsulfonate eingesetzt, eignet sich das erfindungsgemäße Verfahren auch zur Herstellung von Polymeren, die beispielsweise als organische Elektrolumineszenzmaterialien Verwendung finden.

Die Produkte des erfindungsgemäßen Verfahrens sind mehrkernige aromatische Verbindungen, vorzugsweise solche, die sich aus den Formeln (II) und (IV) ergeben. Beispiele für bevorzugte Produkte sind 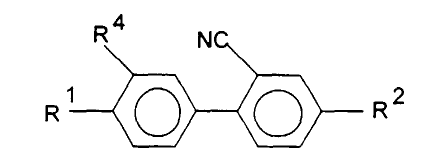 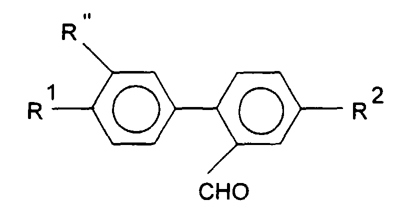 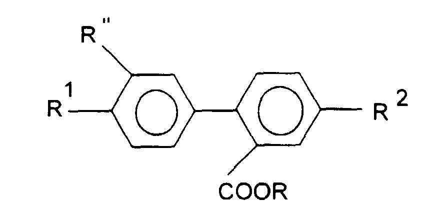 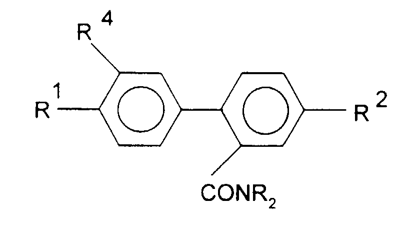 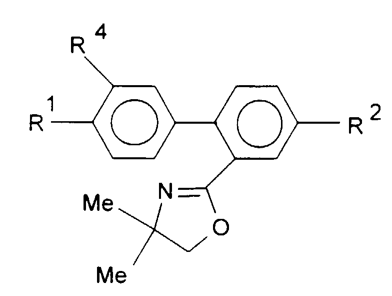 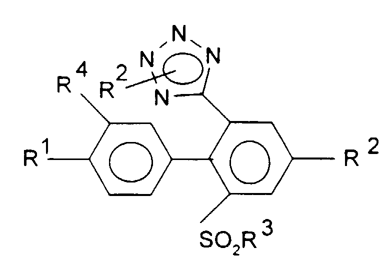 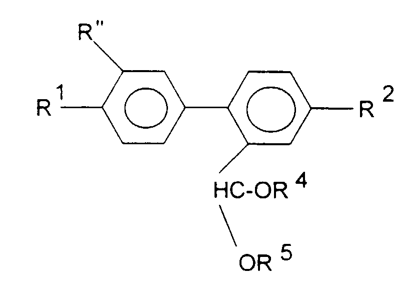 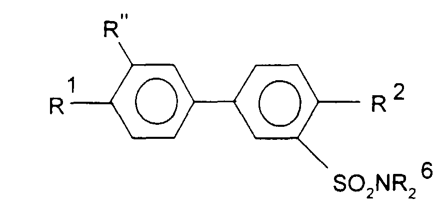 wobei
R¹, R² und R³ Benzyloxy, H, Methyl, Ethyl, Propyl, Butyl, Pentyl, Hexyl, Heptyl, Octyl, Nonyl, Decyl, Undecyl, Dodecyl, Tridecyl, Tetradecyl und Pentadecyl, sowie Methoxy, Ethoxy, Propoxy, Butoxy, Pentoxy, Hexoxy, Heptoxy, Octoxy, Nonoxy, Decoxy, Undecoxy, Dodecoxy, Tridecoxy, Tetradecoxy, Pentadecoxy, CPh₃ und SiMe₂^{t}Bu; R⁴, R⁵ Methyl, Ethyl, Propyl, Butyl, Pentyl, Hexyl, Heptyl, Octyl, Nonyl, Decyl, Undecyl, Dodecl, Tridecyl, Tetradecyl, Pentadecyl oder R⁴ und R⁵ zusammen auch -(CH₂)₂- oder -(CH₂)₃- und R⁴ H oder F bedeuten.

Ein besonders bevorzugtes Produkt ist 2-Cyano-4'-methylbiphenyl.

Die erfindungsgemäß hergestellten Verbindungen eignen sich zum Einsatz als flüssigkristalline Materialien oder können als Zwischenprodukte für die Herstellung weiterer flüssigkristalliner Verbindungen verwendet werden. Des weiteren werden sie als Vorprodukte für Pharmazeutika, Kosmetika, Fungizide, Herbizide, Insektizide, Farbstoffe, Detergenzien und Polymere, einschließlich von Zusatzstoffen derselben, eingesetzt.

Erfindungsgemäß hergestellte Verbindungen, wie sie beispielsweise durch die obigen Formeln wiedergegeben werden, sind insbesondere wertvolle Vorstufen für Angiotensin II Inhibitoren (siehe z.B. Drugs of the Future 18 (1993) 428-432).

Die vorliegende Erfindung soll durch die nachfolgend beschriebenen Beispiele näher erläutert werden, ohne sie dadurch zu begrenzen.

### Beispiele

Katalysatorpräparation:

### K.1

0,388 g Palladium(II)chlorid und 0,54 g Natriumacetat werden in 24 ml DMSO gelöst. Man rührt 30 Minuten bei Raumtemperatur. Anschließend versetzt man mit 14,6 ml TPPTS/H₂O-Lösung (0,6 mol/l) und rührt 30 Minuten nach.

### K.2

0,388 g Palladium(II)chlorid und 0,54 g Natriumacetat werden in 24 ml Ethylenglykol gelöst. Man rührt 30 Minuten bei Raumtemperatur nach. Anschließend versetzt man mit 14,6 ml TPPTS/H₂O-Lösung (0,6 mol/l) und rührt 30 Minuten nach.

### K.3

0,388 g Palladium(II)chlorid und 14,6 ml TPPTS/H₂O-Lösung (0,6 mol/l) werden 60 Minunten bei Raumtemperatur gerührt. Man erhält eine gelbe Reaktionslösung von

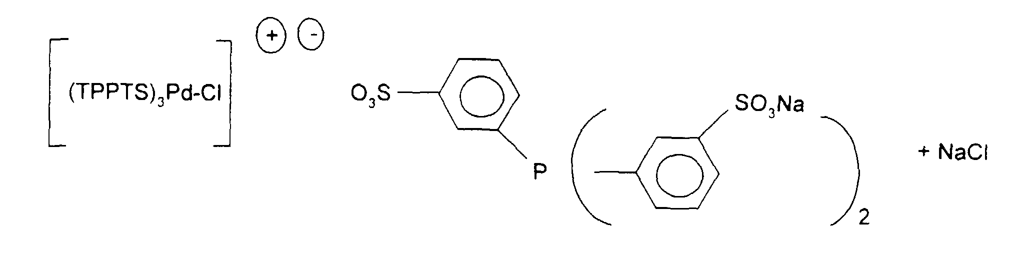

### K.3.1.

1,069 g Tetrachloropalladiumsäure (20 Gew.-% Palladium in Wasser) werden mit 24 ml Wasser verdünnt und anschließend mit 14,6 ml einer 0,6 molaren TPPTS/H₂O-Lösung versetzt. Man rührt 30 Minuten nach.

### K.4

0,388 g Palladium(II)chlorid und 0,33 g Kaliumchlorid werden in 10 ml Wasser gelöst. Man versetzt mit 14,6 ml TPPTS/H₂O-Lösung (0,6 mol/l).

### B. Kupplungsreaktionen

### Beispiel 1

15 g 2-Chlorbenzonitril, 14,8 g p-Toluolboronsäure und 28,9 g Natriumcarbonat wurden in 40 ml Glykol und 10 ml Wasser auf 120°C erhitzt. Bei 80°C gab man 0,1 Mol% einer nach K.1 hergestellten Katalysatorlösung zu. Nach beendeter Reaktion wurde mit 50 ml Xylol versetzt und die organische Phase abgetrennt. Durch Destillation erhielt man 19 g 2-Cyano-4'-methyl-biphenyl (Sdp. 140°C/mbar).

### Beispiel 2

15 g 2-Chlorbenzonitril, 14,8 g p-Toluolboronsäure und 28,9 g Natriumcarbonat wurden in 40 ml Glykol und 10 ml Wasser auf 120°C erhitzt. Bei 80°C gab man 0,1 Mol% einer nach K.2 hergestellten Katalysatorlösung zu. Nach beendeter Reaktion wurde mit 50 ml Xylol versetzt und die organische Phase abgetrennt. Durch Destillation erhielt man 18,5 g 2-Cyano-4'-methyl-biphenyl (Sdp. 140°C/mbar).

### Beispiel 3

15 g 2-Chlorbenzonitril, 14,8 g p-Toluolboronsäure und 28,9 g Natriumcarbonat wurden in 40 ml Glykol und 10 ml Wasser auf 120°C erhitzt. Bei 80°C gab man 0,1 Mol% einer nach K.3 hergestellten Katalysatorlösung zu. Nach beendeter Reaktion wurde mit 50 ml Xylol versetzt und die organische Phase abgetrennt. Durch Destillation erhielt man 18,7 g 2-Cyano-4'-methyl-biphenyl (Sdp. 140°C/mbar).

### Beispiel 4

15 g 2-Chlorbenzonitril, 14,8 g p-Toluolboronsäure und 28,9 g Natriumcarbonat wurden in 40 ml Glykol und 10 ml Wasser auf 120°C erhitzt. Bei 80°C gab man 0,1 Mol% einer nach K.4 hergestellten Katalysatorlösung zu. Nach beendeter Reaktion wurde mit 50 ml Xylol versetzt und die organische Phase abgetrennt. Durch Destillation erhielt man 18,1 g 2-Cyano-4'-methyl-biphenyl (Sdp. 140°C/mbar).

### Beispiel 5

15 g 2-Chlorbenzonitril, 14,8 g p-Toluolboronsäure und 28,9 g Natriumcarbonat wurden in 40 ml Glykol und 10 ml Wasser auf 120°C erhitzt. Bei 80°C gab man 0,1 Mol% einer nach K.3.1 hergestellten Katalysatorlösung zu. Nach beendeter Reaktion wurde mit 50 ml Xylol versetzt und die organische Phase abgetrennt. Durch Destillation erhielt man 19 g 2-Cyano-4'-methyl-biphenyl (Sdp. 140°C/mbar).

### Beispiel 6

15 g 2-Chlorbenzonitril, 14,8 g p-Toluolboronsäure und 12 g Natriumcarbonat wurden in 40 ml Glykol und 10 ml Wasser auf 120°C erhitzt. Bei 80°C gab man 0,1 Mol% einer nach K.1 hergestellten Katalysatorlösung zu. Nach beendeter Reaktion wurde mit 50 ml Xylol versetzt und die organische Phase abgetrennt. Durch Destillation erhielt man 18,5 g 2-Cyano-4'-methyl-biphenyl (Sdp. 140°C/mbar).

### Beispiel 7

15 g 2-Chlorbenzonitril, 14,8 g p-Toluolboronsäure und 12 g Natriumcarbonat wurden in 40 ml Glykol und 10 ml Wasser auf 120°C erhitzt. Bei 80°C gab man 0,1 Mol% einer nach K.3.1 hergestellten Katalysatorlösung zu. Nach beendeter Reaktion wurde mit 50 ml Xylol versetzt und die organische Phase abgetrennt. Durch Destillation erhielt man 18,7 g 2-Cyano-4'-methyl-biphenyl (Sdp. 140°C/mbar).

### Beispiel 8

15 g 2-Chlorbenzonitril, 14,8 g p-Toluolboronsäure und 12 g Natriumcarbonat wurden in 40 ml Glykol und 10 ml Wasser auf 120°C erhitzt. Bei 80°C gab man 0,1 Mol% einer nach K.2 hergestellten Katalysatorlösung zu. Nach beendeter Reaktion wurde mit 50 ml Xylol versetzt und die organische Phase abgetrennt. Durch Destillation erhielt man 18,1 g 2-Cyano-4'-methyl-biphenyl (Sdp. 140°C/mbar).

### Beispiel 9

15 g 2-Chlorbenzonitril, 14,8 g p-Toluolboronsäure und 28,9 g Natriumcarbonat wurden mit 50 ml p-Xylol, 40 ml Glykol und 10 ml Wasser auf 120°C erhitzt. Bei 80°C gab man eine Mischung aus 24,7 mg Palladiumacetat und 0,55 ml TPPTS/H₂O-Lösung (0,6 Molar) in 2,5 ml DMSO zu.

Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 18,9 g 2-Cyano-4'-methylbiphenyl.

### Beispiel 10

15 g 2-Chlorbenzonitril, 14,8 g p-Toluolboronsäure und 28,9 g Natriumcarbonat wurden mit 50 ml p-Xylol 40 ml Glycerin und 10 ml Wasser auf 120°C erhitzt. Bei 80°C gab man eine Mischung aus 24,7 mg Palladiumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 mi Wasser gewaschen und anschließend über Natriumsulfat getrocknet. Kristallisation aus n-Heptan ergab 18,5 g 2-Cyano-4'-methylbiphenyl.

### Beispiel 11

15 g 2-Chlorbenzonitril, 14,8 g p-Toluolboronsäure und 28,9 g Natriumcarbonat wurden mit 50 ml p-Xylol, 40 ml Triethylenglykol und 10 ml Wasser auf 120°C erhitzt. Bei 80°C gab man eine Mischung aus 24,7 mg Palladiumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 17,4 g 2-Cyano-4'-methylbiphenyl.

### Beispiel 12

15 g 2-Chlorbenzonitril, 14,8 g p-Toluolboronsäure und 28,9 g Natriumcarbonat wurden mit 50 ml p-Xylol, 40 ml Diethylenglykol und 10 ml Wasser auf 120°C erhitzt. Bei 80°C gab man eine Mischung aus 24,7 mg Palladiumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 18,2 g 2-Cyano-4'-methylbiphenyl.

### Beispiel 13

15 g 2-Chlorbenzonitril, 14,8 g p-Toluolboronsäure und 28,9 g Natriumcarbonat wurden mit 50 ml p-Xylol, 40 ml Diethanolamin und 10 ml Wasser auf 120°C erhitzt. Bei 80°C gab man eine Mischung aus 24,7 mg Palladiumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 17,5 g 2-Cyano-4'-methylbiphenyl.

### Beispiel 14

15 g 2-Chlorbenzonitril, 14,8 g p-Toluolboronsäure und 28,9 g Natriumcarbonat wurden mit 50 ml p-Xylol, 40 ml Triethanolamin und 10 ml Wasser auf 120°C erhitzt. Bei 80°C gab man eine Mischung aus 24,7 mg Palladiumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 17,8 g 2-Cyano-4'-methylbiphenyl.

### Beispiel 15

15 g 2-Chlorbenzonitril, 14,8 g p-Toluolboronsäure und 28,9 g Natriumcarbonat wurden mit 50 ml p-Xylol, 40 ml Glykol und 10 ml Wasser auf 120°C erhitzt. Bei 80°C gab man eine Mischung aus 38,66 mg Palladium-II-chlorid und 1,1 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 18,5 g 2-Cyano-4'-methylbiphenyl.

### Beispiel 22

15 g 2-Chlorbenzonitril, 14,8 g p-Toluolboronsäure und 28,9 g Natriumcarbonat wurden mit 50 ml p-Xylol, 40 ml Glycerin und 10 ml Wasser auf 120°C erhitzt. Bei 80°C gab man eine Mischung aus 38,66 mg Palladium-II-chlorid und 1,1 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 18,2 g 2-Cyano-4'-methylbiphenyl.

### Beispiel 23

15 g 2-Chlorbenzonitril, 14,8 g p-Toluolboronsäure und 28,9 g Natriumcarbonat wurden mit 50 ml p-Xylol, 40 ml Triethylenglykol und 10 ml Wasser auf 120°C erhitzt. Bei 80°C gab man eine Mischung aus 38,66 mg Palladium-II-chlorid und 1,1 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 17,4 g 2-Cyano-4'-methylbiphenyl.

### Beispiel 24

15 g 2-Chlorbenzonitril, 14,8 g p-Toluolboronsäure und 28,9 g Natriumcarbonat wurden mit 50 ml p-Xylol, 40 ml Diethylenglykol und 10 ml Wasser auf 120°C erhitzt. Bei 80°C gab man eine Mischung aus 38,66 mg Palladium-II-chlorid und 1,1 ml TPPTS/H₂0-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 17,1 g 2-Cyano-4'-methylbiphenyl.

### Beispiel 25

15 g 2-Chlorbenzonitril, 14,8 g p-Toluolboronsäure und 28,9 g Natriumcarbonat wurden mit 50 ml p-Xylol, 40 ml Diethanolamin und 10 ml Wasser auf 120°C erhitzt. Bei 80°C gab man eine Mischung aus 38,66 mg Palladium-II-chlorid und 1,1 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 17,5 g 2-Cyano-4'-methylbiphenyl.

### Beispiel 26

15 g 2-Chlorbenzonitril, 14,8 g p-Toluolboronsäure und 28,9 g Natriumcarbonat wurden mit 50 ml p-Xylol, 40 ml Triethanolamin und 10 ml Wasser auf 120°C erhitzt. Bei 80°C gab man eine Mischung aus 38,66 mg Palladium-II-chlorid und 1,1 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 17,8 g 2-Cyano-4'-methylbiphenyl.

### Beispiel 33

15 g 2-Chlorbenzonitril, 14,8 g p-Toluolboronsäure und 28,9 g Natriumcarbonat wurden mit 50 ml p-Xylol, 40 ml Glykol und 10 ml Wasser auf 120°C erhitzt. Bei 80°C gab man 19,3 mg Palladiumchlorid, 17,9 mg Natriumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 mi Toluol gewaschen. Die vereinigten organischen Phasen wurden mit 20 ml Wasser gewaschen und anschließend über Natriumsulfat getrocknet. Kristallisation aus n-Heptan ergab 18,7 g 2-Cyano-4'-methylbiphenyl.

### Beispiel 34

15 g 2-Chlorbenzonitril, 14,8 g p-Toluolboronsäure und 28,9 g Natriumcarbonat werden mit 50 ml p-Xylol, 40 ml Glycerin und 10 ml Wasser auf 120°C erhitzt. Bei 80°C gab man eine Mischung aus 19,3 mg Palladiumchlorid, 17,9 mg Natriumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 18,3 g 2-Cyano-4'-methylbiphenyl.

### Beispiel 35

15 g 2-Chlorbenzonitril, 14,8 g p-Toluolboronsäure und 28,9 g Natriumcarbonat wurden mit 50 ml p-Xylol, 40 ml Triethylenglykol und 10 ml Wasser auf 120°C erhitzt. Bei 80°C gab man eine Mischung aus 19,3 mg Palladiumchlorid, 17,9 mg Natriumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 17,4 g 2-Cyano-4'-methylbiphenyl.

### Beispiel 36

15 g 2-Chlorbenzonitril, 14,8 g p-Toluolboronsäure und 28,9 g Natriumcarbonat wurden mit 50 ml p-Xylol, 40 ml Diethylenglykol und 10 ml Wasser auf 120°C erhitzt. Bei 80°C gab man eine Mischung aus 19,3 mg Palladiumchlorid, 17,9 mg Natriumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 18,3 g 2-Cyano-4'-methylbiphenyl.

### Beispiel 37

15 g 2-Chlorbenzonitril, 14,8 g p-Toluolboronsäure und 28,9 g Natriumcarbonat wurden mit 50 ml p-Xylol, 40 ml Diethanolamin und 10 ml Wasser auf 120°C erhitzt. Bei 80°C gab man eine Mischung aus 19,3 mg Palladiumchlorid, 17,9 mg Natriumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 17,5 g 2-Cyano-4'-methylbiphenyl.

### Beispiel 38

15 g 2-Chlorbenzonitril, 14,8 g p-Toluolboronsäure und 28,9 g Natriumcarbonat wurden mit 50 ml p-Xylol, 40 ml Triethanolamin und 10 ml Wasser auf 120°C erhitzt. Bei 80°C gab man 19,3 mg Palladiumchlorid, 17,9 mg Natriumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Toluol gewaschen. Die vereinigten organischen Phasen wurden mit 20 ml Wasser gewaschen und anschließend über Natriumsulfat getrocknet. Kristallisation aus n-Heptan ergab 17,8 g 2-Cyano-4'-methylbiphenyl.

### Beispiel 39

15 g 2-Chlorbenzonitril, 14,8 g p-Toluolboronsäure und 28,9 g Natriumcarbonat wurden mit 50 ml p-Xylol, 40 ml DMSO und 10 ml Wasser auf 120°C erhitzt. Bei 80°C gab man eine Mischung aus 24,7 mg Palladiumacetat (II) und 0,55 ml TPPTS/H₂O (0,6 Molar) in 2,5 ml DMSO zu.

Nach beender Reaktion wurden die Phasen getrennt. Die wäßrige Phase wurde mit 50 ml Xylol gewaschen. Die vereinigten organischen Phasen wurden mit 20 ml Wasser gewaschen und anschließend über Natriumsulfat getrocknet. Das Lösungsmittel wurde eingedampft und der Rückstand aus n-Heptan kristallisiert. Ausbeute: 18,6 g (88 % d.Th.) 2-Cyano-4'-methylbiphenyl.

### Beispiel 40

15 g 2-Chlorbenzonitril, 14,8 g p-Toluolboronsäure und 29,8 g Natriumcarbonat wurden mit 50 ml p-Xylol, 40 ml Sulfolan und 10 ml Wasser auf 120°C erhitzt. Bei 80°C gab man eine Mischung aus 24,7 mg Palladiumacetat und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Xylol gewaschen. Die vereinigten organischen Phasen wurden mit 20 ml Wasser gewaschen und anschließend über Natriumsulfat getrocknet. Das Lösungsmittel wurde eingedampft und der Rückstand aus n-Heptan kristallisiert. Ausbeute: 19,2 g (91 % d.Th.) 2-Cyano-4'-methylbiphenyl.

### Beispiel 41

15 g 2-Chlorbenzonitril, 14,8 g p-Toluolboronsäure und 28,9 g Natriumcarbonat wurden mit 50 ml p-Xylol, 40 ml DMSO und 10 ml Wasser auf 120°C erhitzt. Bei 80°C ergab man eine Mischung aus 38,66 mg Palladium-II-chlorid und 1,1 ml TPPTS/H₂O-Lösung (0,6 Molar) in 2,5 ml DMSO zu.
Nach beendeter Reaktion wurden die Phasen getrennt. Die wäßrige Phase wurde mit 50 ml Xylol gewaschen. Die vereinigten organischen Phasen wurden mit 20 ml Wasser gewaschen und anschließend über Natriumsulfat getrocknet. Das Lösungsmittel wurde eingedampft und der Rückstand aus n-Heptan kristallisiert. Ausbeute: 18,2 g (86 % d.th.) 2-Cyano-4'-methylbiphenyl.

### Beispiel 42

15 g 2-Chlorbenzonitril, 14,8 g p-Toluolboronsäure und 28,9 g Natriumcarbonat wurden mit 50 ml p-Xylol, 40 ml Sulfolan und 10 ml Wasser auf 120°C erhitzt. Bei 80°C gab man eine Mischung aus 38,66 mg Palladium-II-chlorid und 1,1 ml TPPTS/H₂O-Lösung (0,6 Molar) in 2,5 ml DMSO zu.
Nach beendeter Reaktion wurden die Phasen getrennt. Die wäßrige Phase wurde mit 50 ml Xylol gewaschen. Die vereinigten Phasen wurden mit 20 ml Wasser gewaschen und anschließend über Natriumsulfat getrocknet. Das Lösungsmittel wurde eingedampft und der Rückstand aus n-Heptan kristallisiert. Ausbeute: 18,4 g (87 % d.Th.) 2-Cyano-4'-methylbiphenyl.

### Beispiel 43

15 g 2-Chlorbenzonitril, 14,8 g p-Toluolboronsäure und 28,9 g Natriumcarbonat wurden mit 50 ml p-Xylol, 40 ml DMSO und 10 ml Wasser auf 120°C erhitzt. Bei 80°C gab man eine Mischung aus 19,3 mg Palladium-II-chlorid, 17,9 mg Natriumacetat und 0,55 ml TPPTS/H₂O-Lösung (0,6 Molar) in 2,5 ml DMSO zu. Nach beendeter Reaktion wurden die Phasen getrennt. Die wäßrige Phase wird mit 50 ml Xylol gewaschen. Die vereinigten organischen Phasen wurden mit 20 ml Wasser gewaschen und anschließend über Natriumsulfat getrocknet. Das Lösungsmittel wurde eingedampft und der Rückstand aus n-Heptan kristallisiert. Ausbeute: 18,8 g (89 % d.Th.) 2-Cyano-4'-methylbiphenyl.

### Beispiel 44

15 g 2-Chlorbenzonitril, 14,8 g p-Toluolboronsäure und 28,9 g Natriumcarbonat wurden mit 50 ml p-Xylol, 40 ml Sulfolan und 10 ml Wasser auf 120°C erhitzt. Bei 80°C gab man eine Mischung aus 19,3 mg Palladium-II-chlorid, 17,9 mg Natriumacetat und 0,55 ml TPPTS/H₂O-Lösung (0,6 Molar) in 2,5 ml DMSO zu. Nach beendeter Reaktion wurden die Phasen getrennt. Die wäßrige Phase wurde mit 50 ml Xylol gewaschen. Die vereinigten organischen Phasen wurden mit 20 ml Wasser gewaschen und anschließend über Natriumsulfat getrocknet. Das Lösungsmittel wurde eingedampft und der Rückstand aus n-Heptan kristallisierte Ausbeute: 18,9 g (89,5 % d.Th.) 2-Cyano-4'-methylbiphenyl.

### Beispiel 45

15 g 2-Chlorbenzonitril, 14,8 g p-Toluolboronsäure und 28,9 g Natriumcarbonat wurden mit 50 ml p-Xylol und 40 ml DMSO auf 120°C erhitzt. Bei 80°C gab man eine Mischung aus 24,7 mg Palladiumacetat (II) und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Xylol gewaschen. Die vereinigten organischen Phasen wurden mit 20 ml Wasser gewaschen und anschließend über Natriumsulfat getrocknet. Das Lösungsmittel wurde eingedampft und der Rückstand aus n-Heptan kristallisisert. Ausbeute: 18,0 g (85 % d.Th.) 2-Cyano-4'-methylbiphenyl.

### Beispiel 46

15 g 2-Chlorbenzonitril, 14,8 g p-Toluolboronsäure und 28,9 g Natriumcarbonat wurden mit 50 ml p-Xylol und 40 ml Sulfolan auf 120°C erhitzt. Bei 80°C gab man eine Mischung aus 24,7 mg Palladiumacetat (II) und 0,55 ml TPPTS/H₂O-Lösung (0,6 Molar) in 2,5 ml DMSO zu.
Nach beendeter Reaktion wurden die Phasen getrennt. Die wäßrige Phase wurde mit 50 ml Xylol gewaschen. Die vereinigten organischen Phasen wurden mit 20 ml Wasser gewaschen und anschließend über Natriumsulfat getrocknet. Das Lösungsmittel wurde eingedampft und der Rückstand aus n-Heptan kristallisiert. Ausbeute: 18,2 g (86 % d.Th.) 2-Cyano-4'-methylbiphenyl.

### Vergleichsbeispiel

15 g 2-Chlorbenzonitril, 14,8 g p-Toluolboronsäure und 28,9 g Natriumcarbonat wurden mit 50 ml p-Xylol und 40 ml DMF auf 120°C erhitzt. Bei 80°C gab man eine Mischung aus 24,7 mg Palladiumacetat (II) und 0,55 ml TPPTS/H₂O-Lösung (0,6 Molar) in 2,5 ml DMF.
Nach beendeter Reaktion wurden die Phasen getrennt. Die wäßrige Phase wurde mit 50 ml Xylol gewaschen. Die vereinigten organischen Phasen wurden mit 20 ml Wasser gewaschen und anschließend über Natriumsulfat getrocknet. Das Lösungsmittel wurde eingedampft und der Rückstand aus n-Heptan kristallisiert. Ausbeute: 1,06 g (5 % d.Th.) 2-Cyano-4'-methylbiphenyl.

### Beispiel 47

### Kreuzkupplung von 2-Chlorbenzonitril mit 4-Toluolboronsäure

Zur Herstellung des Katalysators werden 38,8 mg (0,219 mmol) Palladium-(II)-chlorid und 54,0 mg (0,657 mmol) Natriumacetat in 2,4 ml DMSO unter Argonatmosphäre 30 min bei 23°C gerührt. Anschließend werden 1,99 ml (0,875 mmol) einer 0,44 molaren wäßrigen Lösung von Natrium-4-diphenylphosphinophenyl-phosphinat, hergestellt wie unten angegeben zugegeben und die Suspension wird weitere 30 min bei 23°C gerührt.
Unter Argonatmosphäre werden 30,0 g (0,2181 mol) 2-Chlorbenzonitril, 32,6 g (0,240 mol) 4-Toluolboronsäure und 16,2 g (70 mol%) Natriumcarbonat in 120 ml Ethylenglycol verrührt. Man gibt 20 ml Wasser zu und erwärmt auf 80°C. Nun wird die oben beschriebene Katalysator-Suspension zugegeben und man erhitzt 5 Stunden unter Rückfluß.
Bei 23°C wird die Mischung mit 100 ml Essigester versetzt. Die organische Phase wird abgetrennt, am Rotationsverdampfer eingeengt und im Vakuum fraktioniert destilliert. Man erhält 31,6 g (75 % d. Th.) 2-Cyano-4'-methyl-biphenyl (Kp. 140°C/1,0 mbar; Fp. 50°C).

### Herstellung des Katalysators:

### a) 4-Fluorphenyl-methyl-phosphinsäureisobutylester

Eine Mischung aus 50,0 g (289 mmol) 4-Bromfluorbenzol, 43,3 g (318 mmol) Methanphosphonigsäureisobutylester, 43,8 ml (318 mmol) Triethylamin, 1,64 g (0,29 mmol, 1 mol-%) Palladium-bis-(dibenzylidenaceton) und 1,50 g (0,58 mmol, 2 mol-%) Triphenylphosphan wurden unter Inertgasatmosphäre 20 Stunden bei 100°C erhitzt. Bei 23°C filtrierte man vom entstandenen Ammoniumsalz ab und engte das Filtrat im Vakuum ein. Nach fraktionierter Destillation im Vakuum erhielt man 54,60 g (83 % d.Th.) 4-Fluorphenyl-methylphosphinsäureisobutylester mit einem Siedepunkt von 83°C/0,08 mbar.

### b) (4-Diphenylphosphino-phenyl)-methyl-phosphinsäureisobutylester

Zu einer Lösung von 21,90 g (95 mmol) 4-Fluorphenyl-methylphosphinsäureisobutylester in 150 ml THF wurden bei -5°C 200 ml (100 mmol) Kaliumdiphenylphosphid-Lösung in THF (Hersteller: Aldrich) getropft. Nach 20 Stunden Rühren bei 23°C wurde die Mischung durch Zugabe von 250 ml entgastem Wasser und 15 min Rühren hydrolysiert. Nach Extraktion mit Ethylacetat, Trocknen der organischen Phase über Na₂SO₄, Einengen am Rotationsverdampfer und Trocknen im Vakuum erhielt man 37,0 g (4-Diphenylphosphino-phenyl)-methyl-phosphinsäureisobutylester in Form eines gelben Öls.

### c) (4-Diphenylphosphino-phenyl)-methyl-phosphinsäure

Zu einer Lösung von 34,52 g (87 mmol) (4-Diphenylphosphino-phenyl)-methylphosphinsäureisobutylester in 50 ml THF wurden bei 23°C 8,70 g (217,5 mmol) NaOH in 40 ml Wasser getropft. Nach 6 Stunden Erhitzen unter Rückfluß tropfte man bei 23°C 18,1 ml (218 mmol) konzentrierte Salzsäure zu, rührt 10 min bei 23°C und engte am Rotationsverdampfer vollständig ein. Der Rückstand wird in Methylenchlorid aufgenommen, die Lösung wurde über Na₂SO₄ getrocknet und vollständig eingeengt. Nach Trocknen im Vakuum erhiet man 28,3 g (4-Diphenylphosphino-phenyl)-methyl-phosphinsäure (90 % der Theorie) in Form eines blaßgelben Feststoffes mit Fp. 40°C.

### d) Na-(4-Diphenylphosphino-phenyl)-methyl-phosphinat

15,0 g (44,1 mmol) (4-Diphenylphosphino-phenyl)-methyl-phosphinsäure wurden mit 100 ml einer 3,7 gew.-%igen Natriumhydrogencarbonat-Lösung (44,1 mmol NaHCO₃) versetzt und bei Raumtemperatur solange gerührt, bis die CO₂-Entwicklung beendet war. Die wäßrige Lösung wurde im Vakuum vollständig eingeengt und getrocknet. Man erhielt 15,6 g (97 % d.Th.)
Na-(4-Diphenylphosphino-phenyl)-methyl-phosphinat.
Löslichkeit in Wasser: 590 g/l

## Patentansprüche

1. Verfahren zur Herstellung mehrkerniger aromatischer Verbindungen, dadurch gekennzeichnet, daß man
a) eine aromatische Borverbindung mit
b) einer aromatischen Halogenverbindung oder einem aromatischen Perfluoralkylsulfonat in Gegenwart
c) einer Base aus der Gruppe der Alkali- und Erdalkalimetallhydroxide, Alkali- und Erdalkalicarbonate, Alkalimetallhydrogencarbonate, Alkali- und Erdalkalimetallacetate, Alkali- und Erdalkalimetallalkoholate, sowie primäre, sekundäre und tertiäre Amine,
d) einer Nickel- oder Palladiumverbindung als Katalysator,
e) eines phosphorhaltigen Liganden und
f) eines mehrwertigen Alkohols, Sulfoxids oder Sulfons
umsetzt.

2. Verfahren nach Anspruch 1, dadurch gekennzeichnet, daß der mehrwertige Alkohol, das Sulfoxid oder Sulfon wasserlöslich sind.

3. Verfahren nach Anspruch 2, gekennzeichnet durch einen mehrwertigen wasserlöslichen Alkohol aus der Gruppe Glykole, Glycerin, Oligoglyceride, die auch teilverestert sein können, Di-, Tri- und Tetraethylenglykol oder auch Polyethylenglykole der allgemeinen Formel (I), 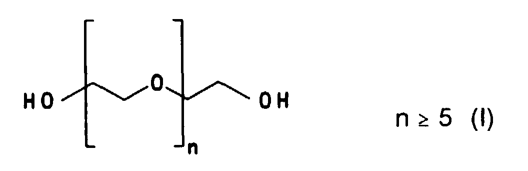 mehrwertige Alkan- oder Alkenole, mehrwertige Cycloalkanole, mehrwertige, Arylgruppen enthaltende Alkanole, mehrwertige Aminoalkohole, mehrwertige Iminoalkohole.

4. Verfahren nach Anspruch 1 oder 2, dadurch gekennzeichnet, daß ein Sulfoxid oder Sulfon der allgemeinen Formel (II) verwendet wird, 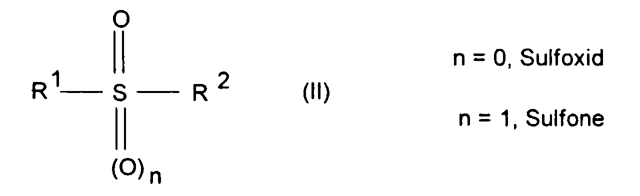 wobei
R¹, R² aliphatische oder aromatische Kohlenwasserstoffe sind, die gegebenenfalls substituiert oder miteinander verknüpft sein können.

5. Verfahren nach einem oder mehreren der Ansprüche 1 bis 4, dadurch gekennzeichnet, daß ein phosphorhaltiger Ligand aus der Gruppe Tri-n-alkylphosphane, Triarylphosphane, Dialkylarylphosphane, Alkyldiarylphosphane und Heteroarylphosphane, wobei die drei Substituenten am Phosphor gleich oder verschieden, chiral oder achiral sein können und wobei einer oder mehrere der Substituenten die Phosphorgruppen mehrerer Phosphane verknüpfen können und wobei ein Teil dieser Verknüpfung auch ein oder mehrere Metallatome sein können, Phosphite, Phosphinigsäureester und Phosphonigsäureester, Phosphole, Dibenzophosphole und Phosphoratome enthaltende cyclische bzw. oligo- und polycyclische Verbindungen eingesetzt wird.

6. Verfahren nach Anspruch 5, dadurch gekennzeichnet, daß der phosphorhaltige Ligand wasserlöslich ist.

7. Verfahren nach einem oder mehreren der Ansprüche 1 bis 6, dadurch gekennzeichnet, daß eine aromatische Borverbindung der Formel (II)
Aryl―BQ₁Q₂ (II)
worin
Aryl ein aromatischer Rest ist und
Q₁, Q₂ gleich oder verschieden -OH, C₁-C₄-Alkoxy, C₁-C₄-Alkyl, Phenyl, das gegebenenfalls durch C₁-C₄-Alkyl, C₁-C₄-Alkoxy oder Halogen substituiert sein kann, oder Halogen bedeuten oder Q₁ und Q₂ zusammen bilden eine C₁-C₄-Alkylendioxy-Gruppe, eine Methylengruppe, die gegebenenfalls durch eine oder zwei C₁-C₄-Alkylgruppen substituiert sein kann, oder Q₁ und Q₂ und das Boratom zusammen sind Teil eines Boroxinrings der Formel (III):

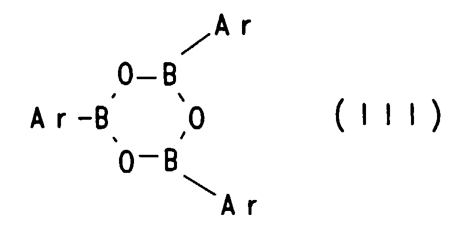

8. Verfahren nach einem oder mehreren der Ansprüche 1 bis 7, dadurch gekennzeichnet, daß eine aromatische Chlorverbindung eingesetzt wird.

## Claims

1. A process for preparing polycyclic aromatic compounds, which comprises reacting
a) an aromatic boron compound with
b) an aromatic halogen compound or an aromatic perfluoroalkylsulfonate in the presence of
c) a base selected from the group consisting of alkali metal and alkaline earth metal hydroxides, alkali metal and alkaline earth metal carbonates, alkali metal hydrogen carbonates, alkali metal and alkaline earth metal acetates, alkali metal and alkaline earth metal alkoxides, and also primary, secondary and tertiary amines,
d) a nickel or palladium compound as catalyst,
e) a phosphorus-containing ligand and
f) a polyhydric alcohol, a sulfoxide or sulfone.

2. The process as claimed in claim 1, wherein the polyhydric alcohol, the sulfoxide or sulfone are water-soluble.

3. The process as claimed in claim 2, wherein the polyhydric water-soluble alcohol is selected from the group consisting of glycols, glycerol, oligoglycerides which can also be partially esterified, diethylene, triethylene and tetraethylene glycols or polyethylene glycols of the formula (I), 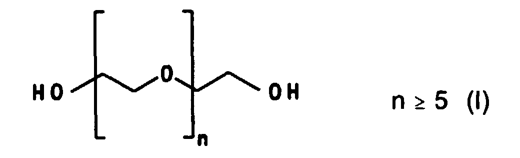 polyhydric alkanols or alkenols, polyhydric cycloalkanols, polyhydric, aryl-containing alkanols, polyhydric aminoalcohols, polyhydric iminoalcohols.

4. The process as claimed in claim 1 or 2, wherein use is made of a sulfoxide or sulfone of the formula (II), 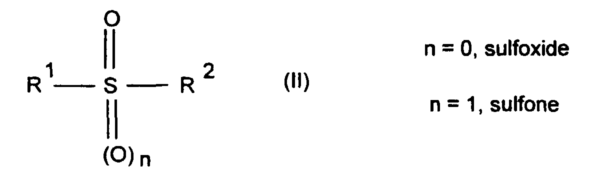 where
R¹, R² are aliphatic or aromatic hydrocarbons which may be unsubstituted, substituted or linked to one another.

5. The process as claimed in one or more of claims 1 to 4, wherein the phosphorus-containing ligand used is selected from the group consisting of tri-n-alkylphosphines, triarylphosphines, dialkylarylphosphines, alkyldiarylphosphines and heteroarylphosphines, where the three substituents on the phosphorus can be identical or different, chiral or achiral and where one or more of the substituents can link the phosphorus groups of a plurality of phosphines and where part of this linkage can also be one or more metal atoms; phosphites, phosphinous esters and phosphonous esters, phosphols, dibenzophosphols and phosphorus-containing cyclic, oligocyclic and polycyclic compounds.

6. The process as claimed in claim 5, wherein the phosphorus-containing ligand is water-soluble.

7. The process as claimed in one or more of claims 1 to 6, wherein the aromatic boron compound used has the formula (II),
Aryl―BQ₁Q₂ (II)
where
Aryl is an aromatic radical and
Q₁, Q₂ are identical or different and are -OH, C₁-C₄-alkoxy, C₁-C₄-alkyl, phenyl which may be unsubstituted or substituted by C₁-C₄-alkyl, C₁-C₄-alkoxy or halogen, or halogen or Q₁ and Q₂ together form a C₁-C₄-alkylenedioxy group, a methylene group which may be unsubstituted or substituted by one or two C₁-C₄-alkyl groups, or Q₁ and Q₂ and the boron atom are together part of a boroxine ring of the formula (III):

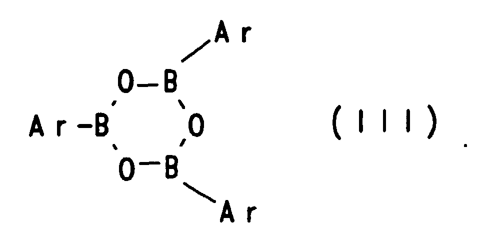

8. The process as claimed in one or more of claims 1 to 7, wherein use is made of an aromatic chlorine compound.

## Revendications

1. Procédé de préparation de composés aromatiques à plusieurs noyaux caractérisé en ce qu'on fait réagir
a) un composé aromatique de bore avec
b) un composé aromatique halogéné ou un perfluroalkylsulfonate aromatique en présence
c) d'une base prise dans le groupe comportant des hydroxydes de métaux alcalins et de métaux alcalino-terreux, des carbonates de métaux alcalins et de métaux alcalino-terreux, des bicarbonates de métaux alcalins, des acétates de métaux alcalins et de métaux alcalino-terreux, des alcoolates de métaux alcalins et de métaux alcalino-terreux, ainsi que des amines primaires, secondaires et tertiaires,
d) d'un composé de nickel ou de palladium en tant que catalyseur,
e) d'un groupe coordonné au phosphore et
f) d'un alcool multifonctionnel, d'un sulfoxyde ou d'une sulfone.

2. Procédé selon la revendication 1, caractérisé en ce que l'alcool multifonctionnel, le sulfoxyde ou la sulfone sont hydrosolubles.

3. Procédé selon la revendication 2, caractérisé par un alcool multifonctionnel hydrosoluble pris dans le groupe comportant des glycols, la glycérine, des oligoglycérides, qui peuvent aussi être partiellement estérifiés, le di-, le tri- et le tétraéthylène glycol ou également des polyéthylèneglycols de formule générale (I) 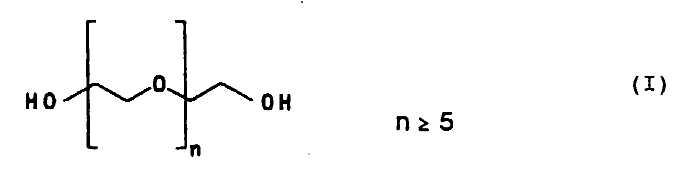 des alcanols ou alcénols multifonctionnels, des cyanoalcanols multifonctionnels, des alcanols multifonctionnels présentant des groupes aryle, des aminoalcools multifonctionnels, des iminoalcools multifonctionnels.

4. Procédé selon la revendication 1 ou 2, caractérisé en ce qu'on emploie un sulfoxyde ou une sulfone de formule générale (II) 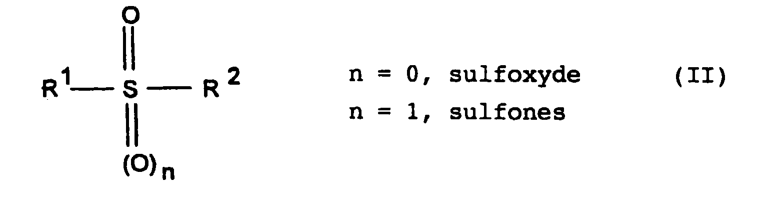 où
R¹, R² représentent des hydrocarbures aliphatiques ou aromatiques, qui peuvent éventuellement être substitués ou reliés entre eux.

5. Procédé selon une ou plusieurs des revendications 1 à 4, caractérisé en ce qu'on emploie un groupe coordonné au phosphore pris dans le groupe comportant des tri-n-alkylphosphanes, des triarylphosphanes, des dialkylarylphosphanes, des alkyldiarylphosphanes et des hétéroarylphosphanes, les trois substituants sur l'atome de phosphore pouvant être identiques ou différents, chiraux ou achiraux et un ou plusieurs des substituants pouvant relier les groupes au phosphore de plusieurs phosphanes et une partie de cette liaison pouvant être formée aussi par un ou plusieurs atomes métalliques, des phosphites, des esters d'acide phosphineux et des esters d'acide phosphoneux, des phosphols, des dibenzophosphols et des composés cycliques, respectivement oligo- et polycycliques présentant des atomes de phosphore.

6. Procédé selon la revendication 5, caractérisé en ce que le groupe coordonné au phosphore est hydrosoluble.

7. Procédé selon une ou plusieurs des revendications 1 à 6, caractérisé en ce qu'un composé aromatique de bore de formule (II)
aryl-BQ₁Q₂ (II)
dans laquelle
aryle représente un reste aromatique et
Q₁, Q₂ sont identiques ou différents et représentent des groupes -OH, alkoxy en C₁-C₄, alkyle en C₁-C₄, phényle, lequel peut éventuellement être substitué par des substituants alkyle en C₁-C₄, alkoxy en C₁-C₄ ou halogène, ou représentent un atome d'halogène, ou Q₁ et Q₂ ensemble forment un groupe (alkylène en C₁-C₄)dioxy, un groupe méthylène, lequel peut éventuellement être substitué par un ou deux groupes alkyle en C₁-C₄, ou Q₁ et Q₂ et l'atome de bore ensemble font partie d'un cycle boroxine de formule (III)

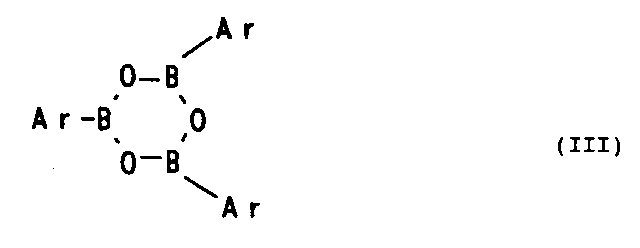

8. Procédé selon une ou plusieurs des revendications 1 à 7, caractérisé en ce qu'on emploie un composé aromatique chloré.
